# EUROPEAN PATENT APPLICATION

(11) **EP 2 036 444 A1**
(43) Date of publication of application: **18.03.2009**
(21) Application number: 08150061.3
(22) Date of filing: 04.01.2008
(51) Int. Cl.: A23L 1/30, A23L 2/02, A23L 1/28, A61K 31/575

(54) **Liquid nutraceutic preparation containing free plant sterols**

(30) Priority: 05.09.2007 EP 07115751
(71) Applicant: Dietetics Pharma S.r.l., 20138 Milano (IT)
(72) Inventor: Angellotti, Carlo, 20138, MILANO (IT)
(74) Representative: Fisauli, Beatrice A. M.

(57) **Abstract**

The present invention relates to a nutraceutical preparation comprising an aqueous preparation containing free plant sterols in the global quantity of 0.5 to 3.0 g/10 ml of water.

The preparation also preferably includes red yeast rice and natural antioxidant compounds, the latter generally being carried by products deriving from fruit and/or vegetables.

The invention also relates to the process for its preparation, and the use of the preparation in foodstuffs to reduce and control the cholesterol level, mainly in the blood.

## Description

The present invention relates to a nutraceutical preparation comprising free plant sterols, and to its preparation process and the use of the preparation in the diet to reduce and control the cholesterol level, mainly in the blood.

In particular, the nutraceutical preparation according to the present invention reduces the cholesterol levels associated with low-density lipoproteins in human blood.

It is well known that cardiovascular disease is very common nowadays, and is a major cause of death, especially among the populations of Western Europe and

North America. Unfortunately, these diseases are also becoming increasingly widespread in the developing countries.

Many factors contribute to the onset of cardiovascular disease, such as a hereditary predisposition, gender, lifestyle factors such as smoking, diet, sedentary lifestyle, age, hypertension and hyperlipidaemia, which includes hypercholesterolaemia. These factors, especially the last two, contribute to the development of atherosclerotic plaques, which are a leading cause of vascular and heart disease.

It is well-know that there is a close relationship between high levels of cholesterol associated with low-density lipoproteins (LDL cholesterol) and an increased risk of heart and coronary disease.

Drugs, foodstuffs and diet products which induce a reduction of LDL cholesterol in the blood have already been developed for some time to reduce this risk.

It is well known that some natural plant substances reduce the plasma cholesterol level following a reduction in total and LDL cholesterol levels. These substances include soya lecithin and plant sterols, for example.

Products containing these natural plant substances are already well known. The only products marketed to date which contain plant sterols that produce said effects are foodstuffs such as yoghurt.

The preparations so far known and marketed present a number of drawbacks.

It is well known that soya lecithin is far less effective than certain plant sterols in combating moderate hypercholesterolaemia, and in particular in reducing the LDL cholesterol level in the plasma.

Products containing plant sterols are therefore preferred to products containing soya lecithin, because they are much more effective. However, said foodstuffs present some disadvantages which limit their use.

First of all, in view of its nature, yoghurt has a short shelf life of only a few days, and also requires low storage temperatures. This requires the provision of suitable (refrigerated) structures for its transport and display to the public. These structures involve higher running costs than preparations which can be stored at ambient temperature.

Moreover, the daily dose of plant sterols which needs to be taken is contained in nearly 100 g of yoghurt. The yoghurt bottles therefore take up a certain amount of space.

In addition, in order to take said daily dose of plant sterols, consumers must eat a food with an energy content of 364 kJ (that is, 87 kcal) which, though not extremely high, is by no means negligible. Finally, this food contains fats, including saturated fats, which must be taken into account in the daily diet balance of persons following a low-fat and/or low-cholesterol diet.

A nutraceutical preparation which does not present the said drawbacks, or presents them to a much lesser extent, is therefore required.

A nutraceutical preparation which contains little or no fat, comprising plant sterols with a cholesterol-reducing action, has now surprisingly been found. The free plant sterols in the nutraceutical preparation according to the present invention are present in water-dispersible form, thus producing a microsuspension in water. This means that there is no need to add a significant quantity of fat to the nutraceutical preparation in order to carry and absorb the plant sterols.

One advantage of the nutraceutical preparation according to the present invention is therefore that the dietary fat intake, and consequently the global calorie intake, is reduced. This is especially advantageous for persons following a low-calorie and, if necessary, a low-fat diet, which is not unusual in individuals suffering from hypercholesterolaemia.

Another advantage is that the almost total absence of fats and micro-organisms, as in yoghurt, means that the nutraceutical preparation according to the present invention does not require low storage temperatures, thus considerably increasing its shelf life, which is 2 years, and reducing the management costs of the product.

A further financial advantage derives from the considerable reduction in volume of the nutraceutical preparation according to the present invention, per daily dose unit of plant sterols, compared with the yoghurt-based product. The preparation according to the invention consists of a microsuspension of only 30 ml, while the volume of the products currently marketed is approximately twice as much.

It is therefore an object of the present invention a nutraceutical preparation comprising, in the same dose unit, an aqueous preparation comprising free plant sterols in the global quantity of 0.5 to 3.0, and preferably 1.0 to 3.0, g/10 ml of water, said plant sterols comprising (percentage in weight):
1) 45-80%, preferably 55-80%, of β-sitosterol, and
2) 8-40%, preferably 10-35%, of campesterol. β-sitosterol and campesterol constitute 70 to 100%, preferably 75 to 100%, and more preferably 80 to 100%, in weight of the total content of the plant sterols in the aqueous preparation of the nutraceutical preparation according to the present invention.

In the present description, the term "sterols" includes both sterols properly so called and derivatives thereof in reduced form called "stanols".

In this description, the term "free" referring to plant sterols means that the hydroxyl group of the sterols is free, and consequently, for example, the sterols are not in esterified form.

In addition to said sterols, other types of plant sterols can also be included, which do not exceed a total of 30%, preferably 25%, and more preferably 20%, by weight of the total weight of the plant sterols.

Specific examples of said further sterols are stigmasterol and brassicasterol.

Specific examples of stanols are sitostanol and campestanol, for example.

The aqueous preparation according to the present invention contains sterols in water-dispersed form.

The water is preferably purified by osmosis.

The nutraceutical preparation according to the present invention preferably comprises further constituents which act independently, simultaneously and/or in synergy with the said free plant sterols, thus increasing the anticholesterol effect of the nutraceutical preparation according to the present invention.

Said aqueous preparation therefore preferably comprises other constituents with said cholesterol-reducing effect, such as "red yeast rice". Said product has been known for a very long time for its cholesterol-lowering capacity and in general for its ability to maintain the serum lipids at optimum levels.

In the present description, "red yeast rice" means the product obtained from fermentation of red rice with various strains of Monascus yeast. Said rice has a monacolin content which is generally equal to or greater than 0.4%. In said aqueous preparation, the red yeast rice with a monacolin content of 0.4% is present in the quantity of between 250 and 350 mg/10 ml of water.

It is obvious that if the red yeast rice contains a monacolin content different from the percentage indicated above, the quantity of dried extract present in the preparation will obviously be varied accordingly, so that the quantity of monacolin in preparation does not exceed 3 mg.

Said red yeast rice is a well-known commercial product; it is generally marketed and used in the form of a powdered dried extract of red rice fermented by one or more strains of Monascus yeast. The strain most commonly used is *Monascus purpureus.* Other strains which could be used are, for example, *Monascus ruber, Monascus Fuliginosus, Monascus Pilosus* and *Monascus Albidus.*

In this description, "unit dose" means a single dose which can be taken by a person and can be rapidly handled and packaged, while remaining a chemically and physically stable unit dose, which includes the active constituents according to the present invention.

The nutraceutical preparation according to the present invention also preferably includes, in the same unit dose, natural antioxidant compounds, such as some vitamins, in particular vitamin A, vitamin C and their precursors, and other natural antioxidants such as polyphenols, flavonoids, etc.

Antioxidant compounds are known to possess cholesterol-lowering effects, because they help to reduce the plasma levels of LDL cholesterol.

Said natural antioxidant compounds are introduced into the nutraceutical preparation according to the invention preferably as products deriving from fruit and/or vegetable processing. For example, the extract, juice, centrifugate, concentrated juice, concentrated flesh or concentrated purée can be used. Said products deriving from fruit and/or vegetable processing are preferably used in wholefood form.

The product deriving from fruit and/or vegetable processing present in the nutraceutical preparation according to the present invention will preferably contain a large amount of antioxidant agents, such as said vitamins; the total antioxidant power of these products, measured in ORAC (Oxigen Radical Absorbance Capacity) units, is preferably at least equal to or greater than 2000 µmoles TE/g, for example between 3500 and 7000 µmoles TE/g.

Products deriving from fruit and vegetables containing large amounts of natural antioxidants, such as citrus fruit, in particular oranges, soft fruits, black grapes and cabbage, are preferred.

The nutraceutical preparation according to the present invention can also include other compounds as well as said constituents, in particular nutrient compounds, come carbohydrates, proteins and small amounts of lipids.

The preparation according to the invention is manufactured by known mixing and homogenisation techniques in a suitable appliance, such as a mixer and/or homogeniser, under the usual conditions.

A possible preparation process involves adding said sterols in water-dispersible form, and optionally red yeast rice, to the water. The various constituents are added to the water in any order. Two or more of said constituents can be premixed together, and the mixture of said constituents is then added to the water.

For example, sterols in water-dispersible form can be added as such individually, or in a mixture of said sterols or in a mixture of said sterols with other compounds, such as said nutrient compounds, which said mixture is in the form of a powder dispersible in water. Mixtures in the form of a powder comprising said free water-dispersible sterols, carbohydrates, proteins, and lipids are available on the market.

Such a process can include a subsequent stage wherein the fruit and/or vegetable juice and/or centrifugate is added to the preparation thus obtained and further mixing or homogenisation is performed.

Each mixing and/or homogenisation stage in such a process is performed under vacuum.

Both the said stages are preferably performed at around ambient temperature or lower, generally between 10° and 30° C, and preferably between 15° and 26° C.

The duration and speed of mixing/homogenisation depends on the type of appliance; for example, at a mixing and/or homogenisation speed ranging between 1100 and 1300 rpm, the mixing and/or homogenisation time is usually 5 to 15 minutes.

Said juice and centrifugate used in the present invention are generally used in the form rehydrated with water purified by osmosis, which is added at the time of use in variable quantities, depending on the desired volume of the corresponding concentrated and subsequently dehydrated product. Said dehydrated products are well-known commercial products, and products with different micronutrient contents, including said antioxidant compounds, are available on the market.

Said aqueous preparation and the nutraceutical preparation according to the present invention can include any other artificial, or preferably natural, constituent habitually used by one skilled in the art in the formulation of aqueous preparations to make non-hydrophilic compounds, such as sterols, and nutraceutical preparations, such as emulsifiers, additives, excipients, preservatives, sweeteners, essences, colorants and optionally other substances, water-dispersible. The nutraceutical preparation according to the present invention contains no probiotic enzymes.

The nutraceutical preparation according to the present invention can be formulated, for example, as a single aqueous preparation or as a kit containing two or more separate liquid preparations.

The nutraceutical preparation according to the present invention should be taken in the approximate amount of one or two unit doses a day, preferably immediately after a meal containing fats.

A non-limiting example of the present invention is set out below.

### EXAMPLE

A microsuspension is prepared in a vacuum mixer with 150 ml turbine, turbodiesel model, by introducing the following constituents in succession:
1) 20 g of juice reconstituted from cloudy concentrated orange juice, which said concentrated juice has a Brix grade of 65° at 20°C and is concentrated 5.9 times,
2) 1400 mg of a composition in powder form comprising 980 mg of the following phytosterols:
   - 690.9 mg (70.5%) of µβ-sitosterol
   - 144.1 mg (14.7%) of campesterol,
   - 27.4 mg (2.8%) of brassicasterol,
   - 9.8 mg (1%) of stigmasterol,
   - 79.4 mg (8.1 %) of sitostanol,
   - 8.8 mg (0.9%) of campestanol, and
   - 19.6 mg (2%) of other sterols,
3) 260 mg of dried extract of red yeast rice with a monacolin K content of 0.4% in weight,
4) 30 mg of potassium sorbate,
5) 3 mg of sodium saccharine,
6) 3 mg of sodium cyclamate,
7) 25 mg of Xanthan gum
8) 1500 mg of sorbitol, and
9) 12.5 ml of water purified by osmosis.

The dried extract of red yeast rice used is a powder with a monacolin content determined by HPLC.

Said reconstituted juice has a Brix grade of 11 at 20° C, and generally has an antioxidant power of between 5000 and 6000 µmoles TE/g.

After the introduction of the first two constituents and after the introduction of each subsequent constituent, mixing is performed at a temperature of approximately 25°C. Mixing takes place at a rotation speed of 1140 rpm, and continues for 1 to 7 minutes, depending on the constituent introduced.

The microsuspension thus obtained is then made up to a volume of 30 ml by adding further osmosis-purified water; after said addition the microsuspension is mixed again, under the same conditions as before. The microsuspension thus produced constitutes a unit dose.

Around 10 days' treatment with one unit dose a day in a patient suffering from moderate hypercholesterolaemia reduces the plasma LDL cholesterol level by approximately 10%.

## Claims

1. A nutraceutical preparation comprising an aqueous preparation comprising free plant sterols in the global quantity of 0.5 to 3.0 g/10 ml of water, said plant sterols comprising (percentage in weight):
1) 45-80% of β-sitosterol and
2) 8-40% of campesterol; said β-sitosterol and campesterol constitute between 70 and 100% of the total weight content of the plant sterols in the aqueous preparation.

2. The nutraceutical preparation according to claim 1, wherein the aqueous preparation comprises free plant sterols in the global quantity of 1.0 to 3.0 g/10 ml of water.

3. The nutraceutical preparation according to claim 1 or 2, wherein the aqueous preparation comprises (percentage in weight):
1) 55-80% β-sitosterol and
2) 10-35% campesterol.

4. The nutraceutical preparation according to claims 1 to 3, wherein the aqueous preparation contains 75 to 100% in weight of β-sitosterol and campesterol as a percentage of the total weight content of the plant sterols in the aqueous preparation.

5. The nutraceutical preparation according to claims 1 to 4, wherein the aqueous preparation comprises 80 to 100% in weight of β-sitosterol and campesterol as a percentage of the total weight content of the plant sterols in the aqueous preparation.

6. The nutraceutical preparation according to claims 1 to 5, wherein the aqueous preparation contains 250 to 350 mg/10 ml of water of a dried extract of red yeast rice fermented by at least one strain of Monascus yeast, said extract having a monacolin content of 0.4%.

7. The nutraceutical preparation according to claims 1 to 6, comprising at least one natural antioxidant compound in the same unit dose.

8. The nutraceutical preparation according to claim 7, wherein the natural antioxidant compound is introduced with a product deriving from fruit and/or vegetable processing.

9. The nutraceutical preparation according to claim 8, wherein the product deriving from fruit and/or vegetable processing has an antioxidant power greater than 2000 µmoles TE/g.

10. The nutraceutical preparation according to claim 8 or 9, wherein the product deriving from fruit and/or vegetable processing is a fruit and/or vegetable juice and/or centrifugate.

11. The nutraceutical preparation according to claims 8 to 10, wherein the product deriving from fruit and/or vegetable processing is obtained from citrus fruit, soft fruits, black grapes or cabbage.

12. The nutraceutical preparation according to claim 11, wherein the fruit juice is orange juice.

13. The nutraceutical preparation according to claims 8 to 12, wherein the juice and centrifugate are wholefood products.

14. A process for manufacturing the nutraceutical preparation according to claims 1 to 5, which involves suspending the sterols in water and then homogenising the preparation thus obtained.

15. A process as according to claim 14 for manufacturing the nutraceutical preparation according to claims 6 and 7, also including a stage at which dried extract of red yeast rice is added before or after the sterols.

16. A process as according to claims 14 and 15 for manufacturing the nutraceutical preparation according to claims 7 to 13, which also includes a stage at which the product deriving from fruit and/or vegetable processing is added and the preparation is then mixed and/or homogenised.

17. The nutraceutical preparation according to claims 1 to 13 , for the treatment of hypercholesterolaemia.
